# EUROPEAN PATENT SPECIFICATION

(11) **EP 2 101 878 B1**
(45) Date of publication and mention of the grant of the patent: **12.05.2010**
(21) Application number: 07847810.4
(22) Date of filing: 04.12.2007
(51) Int. Cl.: A61Q 5/04, A61K 8/20, A61K 8/24, A61K 8/36, A61K 8/365, A61K 8/41, A61K 8/49

(54) **HAIR STRAIGHTENING COMPOSITION**
HAARSTÄRKUNGSZUSAMMENSETZUNG
COMPOSITION DE DÉFRISEMENT DES CHEVEUX

(30) Priority: 16.01.2007 EP 07100579
(43) Date of publication of application: 23.09.2009
(73) Proprietor: Unilever PLC, 100 Victoria Embankment London EC4Y 0DY (GB); Unilever N.V., 3013 AL Rotterdam (NL)
(72) Inventor: CARPENTER, Paul, Merseyside CH63 3JW (GB); KHOSHDEL, Ezat, Merseyside CH63 3JW (GB); TAYLOR, Cheryl, Anne, Merseyside CH63 3JW (GB)
(74) Representative: Tansley, Sally Elizabeth
(86) International application number: PCT/EP2007/063311
(87) International publication number: WO 2008/086914

(56) References cited:
- EP-A- 1 475 076
- EP-A1- 0 368 763
- WO-A-02/085317
- WO-A-2006/068276
- GB-A- 1 187 568

## Description

### Field of the Invention

The present invention relates to a composition and method for straightening hair.

### Background and Prior Art

Products known as "hair relaxers" relax or straighten naturally curly or kinky hair. Hair relaxers may either be applied in a hair salon by a professional or in the home by the individual consumer.

Hair relaxers usually contain an alkaline agent, usually a source of hydroxide ions, to straighten hair. The term "lanthionizing" is used when referring to hair relaxed or straightened by hydroxide ions, as the straightening reaction sequence with hydroxide ions results in lanthionine residue formation.

A key problem with hair relaxers, especially those based on hydroxide ions, is that they damage the hair and so leave the hair treated therewith feeling rough and unconditioned.

WO 02/085317 discloses compositions and methods for lanthionizing keratin fibres using at least one organic nucleophile and at least one hydroxide ion generator.
WO 02/085317 discloses that the organic nucleophile is especially effective when used in a pre-treatment composition.

GB 1 187 568 discloses methods of changing the configuration of the hair by using homocysteine thiolactone.

The present invention relates to hair relaxing systems which mitigate the damage to hair so leaving the hair smooth, soft and easy to comb.

### Description of the Invention

The present invention, in one aspect, provides a hair straightening composition comprising:
i) gamma-thiobutyrolactone;
ii) an acid or salt thereof; and
iii) an amine.

As a variant on the above the invention also relates to a hair straightening kit comprising at least two compositions; the first composition comprising the composition as described above; the second composition comprising hydrogen peroxide.

In another aspect of the invention, there is provided a method for straightening hair comprising applying to the hair a composition described above.

Also described is a method of straightening hair in which the compositions of the kit described above are pre-mixed prior to application.

### Detailed Description of the Invention

### Gamma-thiobutyrolactone

The composition comprises as an essential aspect of the invention gamma-thiobutyrolactone.

The level of gamma-thiobutyrolactone is preferably from 0.1 to 15 wt% of the total composition, more preferably from 0.5 to 10 wt%, most preferably from 1 wt% to 6 wt%.

### Acid

The composition comprises an acid or salt thereof. Preferably the acid is selected from the group consisting of hydrochloric acid, citric acid, acetic acid, phosphoric acid, toluenesulphonic acid or mixtures thereof.

The level of acid is preferably from 0.1 to 15 wt% of the total composition, more preferably from 0.5 to 10 wt%, most preferably from 1 wt% to 6 wt%.

The weight ratio of gamma-thiobutyrolactone to acid is preferably from 3:1 to 1: 3, more preferably from 2:1 to 1:2.

### Amine

The composition of the invention comprises an amine.

Preferred amines are short chain (C1-C10) alkyl amines and/or alkanolamines and/or thioamines. Preferred amines are cysteamine, isopropanolamine, diethanolamine, triethanolamine, 2-amino-2-methyl-1, 3 diol.

It is particularly advantageous for straightening if the amine is monoethanolamine (MEA).

### pH of Composition

The pH of the formulation is preferably from 8 to 10.5, more preferably from 8.5 to 10.

### Other Constituents

Penetration of the composition is aided if after application to the hair, the hair is treated with a solution of oxidising agent, particularly preferred is hydrogen peroxide. The oxidising agent is preferably present from 1 to 20 wt%.

Other constituents, which can be used in the compositions of the invention can be chosen from solvents such as alcohol and water; preservatives; perfumes; UV filters; active hair care agents; plasticizers; anionic, cationic, amphoteric, nonionic, and zwitterionic surfactants; hair conditioning agents such as silicone fluids, fatty esters, fatty alcohol, fatty chain hydrocarbons, emollients, lubricants, and penetrants (such as lanolin compounds), protein hydrolysates, and other protein derivatives; anionic, cationic, amphoteric, nonionic, and zwitterionic polymers; dyes; tints; bleaches; reducing agents; pH adjusting agents; sunscreens; and thickening agents.

Products of the invention may further comprise organic nucleophiles chosen from basic amino acids such as lysine, amines, alcohols and mercaptans and derivatives thereof.

### Product Format

This post treatment composition may be in any form preferably in the form chosen from emulsions, solutions, suspensions, gels, creams, and pastes.

The invention will now be illustrated by the following nonlimiting Examples.

### Examples

### Base composition:

0.35M (3.57%) gamma-thiobutyrolactone
0.44M acid (as in table below)
Liquid monoethanolamine (to pH 9.5)
10% n-ethanol
Water to 100%

The compositions were prepared by adding gamma-thiobutyrolactone to an ethanol water mixture (75 wt% of water) followed by addition of the acid. Finally the monoethanolamine (in the reminder of the water) was added.

### Treatment method:

- Solutions were made and used immediately.
- Approx 1g of hair was immersed in 50ml of solution (50:1 solution to hair).
- This was left for 5 mins, then removed from solution and manually detangled/smoothed straight then immediately returned to solution.
- This was repeated until a total treatment time (after the initial immersion) of 20min (I.e. 4 times).
- Hair was then washed three times with a standard neutralising shampoo (essentially 14:2 SLES:CAPB), each wash consisted of massaging ∼1ml of the shampoo into a 1g hair sample for 30s, followed by rinsing under a running tap for 30s).
- Hair was then left to dry naturally.

### Curvature Measurement:

- Hair curvature was measured using a single-fibre image-analysis technique.

The following acids were added to the base formulation above. Water and 2wt% NaOH were used as a reference control treatment.

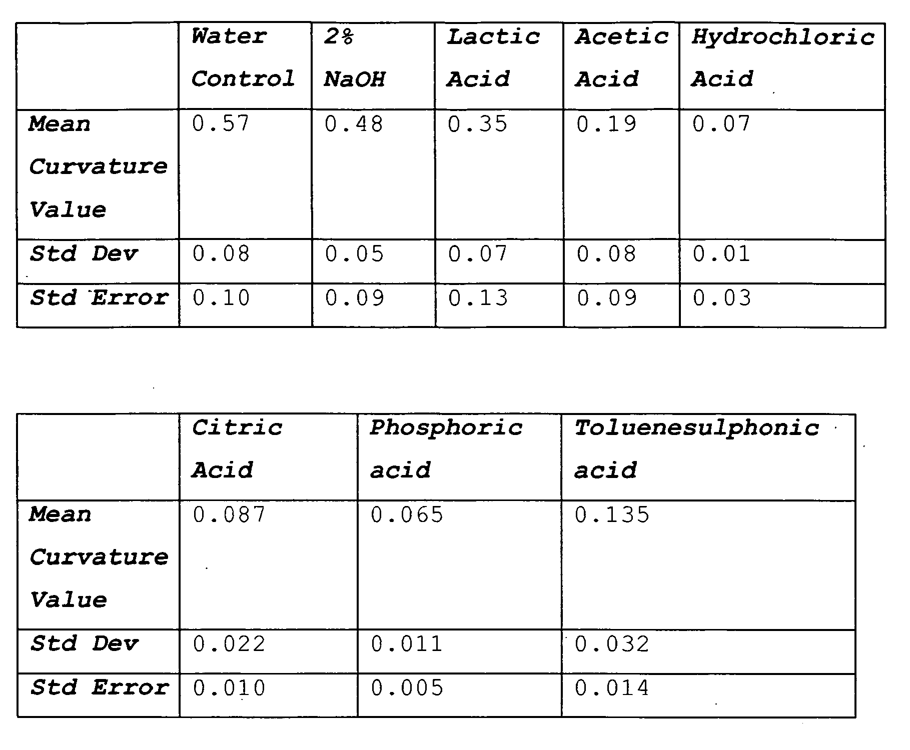

The lower the curvature value the straighter the hair.

## Claims

1. A hair straightening composition comprising:
i) gamma-thiobutyrolactone;
ii) an acid or salt thereof; and
iii) an amine.

2. A hair straightening composition according to claim 1 in which the acid is selected from the group consisting of hydrochloric acid, citric acid, acetic acid, phosphoric acid, toluenesulphonic acid or mixtures thereof.

3. A hair straightening composition according to claim 1 or claim 2 in which the amine is monoethanolamine (MEA) .

4. A hair straightening composition according to any preceding claim in which the level of gamma-thiobutyrolactone is from 1 wt% to 6 wt% of the total composition.

5. A hair straightening composition according to any preceding claim in which the level of acid is from 1 wt% to 6 wt% of the total composition.

6. A hair straightening composition according to any preceding claim in which the weight ratio of gamma-thiobutyrolactone to acid is from 2:1 to 1:2.

7. A hair straightening composition according to any preceding claim in which the pH of the formulation is from 8.5 to 10 at room temperature.

8. A hair straightening kit comprising at least two compositions;
the first composition comprising the composition as described in any of the previous claims;
the second composition comprising hydrogen peroxide.

9. A method for lanthionizing keratin fibres to achieve relaxation of said keratin fibres in which a composition according to any of the preceding claims 1 to 7 is applied to the hair.

## Patentansprüche

1. Haar glättende Zusammensetzung, umfassend:
i) gamma-Thiobutyrolacton;
ii) eine Säure oder ein Salz davon, und
iii) ein Amin.

2. Haar glättende Zusammensetzung gemäß Anspruch 1, wobei die Säure ausgewählt ist aus der Gruppe, bestehend aus Salzsäure, Citronensäure, Essigsäure, Phosphorsäure, Toluolsulfonsäure und Gemischen davon.

3. Haar glättende Zusammensetzung gemäß Anspruch 1 oder Anspruch 2, wobei das Amin Monoethanolamin (MEA) ist.

4. Haar glättende Zusammensetzung gemäß einem vorangehenden Anspruch, wobei die Konzentration an gamma-Thiobutyrolacton 1 Gewichts-% bis 6 Gewichts-% der gesamten Zusammensetzung ist.

5. Haar glättende Zusammensetzung gemäß einem vorangehenden Anspruch, wobei die Konzentration an Säure 1 Gewichts-% bis 6 Gewichts-% der gesamten Zusammensetzung ist.

6. Haar glättende Zusammensetzung gemäß einem vorangehenden Anspruch, wobei das Gewichtsverhältnis von gamma-Thiobutyrolacton zu Säure von 2:1 bis 1:2 ist.

7. Haar glättende Zusammensetzung gemäß einem vorangehenden Anspruch, wobei der pH der Formulierung bei Raumtemperatur 8,5 bis 10 ist.

8. Kit zum Glätten von Haar, umfassend wenigstens zwei Zusammensetzungen, wobei die erste Zusammensetzung die Zusammensetzung, wie sie in einem vorangehenden Anspruch beschrieben ist, umfasst;
die zweite Zusammensetzung Wasserstoffperoxid umfasst.

9. Verfahren zur Lanthionisierung von Keratinfasern zur Erzielung einer Entspannung der Keratinfasern, bei dem eine Zusammensetzung gemäß einem der vorangehenden Ansprüche 1 bis 7 auf das Haar aufgetragen bzw. angewendet wird.

## Revendications

1. Composition lissante pour les cheveux comprenant :
i) la γ-thiobutyrolactone ;
ii) un acide ou un sel de celui-ci ; et
iii) une amine.

2. Composition lissante pour les cheveux selon la revendication 1, dans laquelle l'acide est choisi dans le groupe constitué de l'acide chlorhydrique, l'acide citrique, l'acide acétique, l'acide phosphorique, l'acide toluènesulfonique ou des mélanges de ceux-ci.

3. Composition lissante pour les cheveux selon la revendication 1 ou la revendication 2, dans laquelle l'amine est la monoéthanolamine (MEA).

4. Composition lissante pour les cheveux selon l'une quelconque des revendications précédentes, dans laquelle le taux de gamma-thiobutyrolactone est compris dans la plage allant de 1 % en poids à 6 % en poids de la composition totale.

5. Composition lissante pour les cheveux selon l'une quelconque des revendications précédentes, dans laquelle le taux d'acide est compris dans la plage allant de 1 % en poids à 6 % en poids de la composition totale.

6. Composition lissante pour les cheveux selon l'une quelconque des revendications précédentes, dans laquelle le rapport en poids de la γ-thiobutyrolactone à l'acide est compris dans la plage allant de 2/1 à 1/2.

7. Composition lissante pour les cheveux selon l'une quelconque des revendications précédentes, dans laquelle le pH de la formulation est compris dans la plage allant de 8,5 à 10 à température ambiante.

8. Kit lissant pour les cheveux comprenant au moins deux compositions :
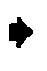 la première composition comprenant la composition décrite dans l'une quelconque des revendications précédentes ;
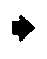 la deuxième composition comprenant du peroxyde d'hydrogène.

9. Procédé de lanthionisation des fibres de kératine pour obtenir un raidissement desdites fibres de kératine, dans lequel une composition selon l'une quelconque des revendications précédentes 1 à 7 est appliquée sur les cheveux.
